# EUROPEAN PATENT APPLICATION

(11) **EP 1 174 027 A1**
(43) Date of publication of application: **23.01.2002**
(21) Application number: 00202562.5
(22) Date of filing: 17.07.2000
(51) Int. Cl.: A01N 37/46, A23B 4/20, A23L 3/3526, A61L 12/14, A61K 7/48, C11D 3/32, A01H 5/00

(54) **Uses of antimicrobial peptides**

(71) Applicant: HOM Consultancy B.V., 2333 CD Leiden (NL)
(72) Inventor: Keijser, Ewald Clemens Raphael Maria, 2343 LB Oegstgeest (NL)
(74) Representative: Wittop Koning, Tom Hugo

(57) **Abstract**

Described are novel uses of antimicrobial peptides or proteins, comprising an amino acid domain, chosen from the group, consisting of the following amino acid sequences: KRLFKKLKFSLRKY, KRLFKKLLFSLRKY, LLLFLLKKRKKRKY, or an amino acid domain sharing at least 40% identity therewith, as active ingredient in an antimicrobial preparation for surface treatment of articles to counteract microbial growth on the said surface, and as additive in human and animal food, hygienic care products, desinfectants, cleaning agents and biocides. Further a transgenic plant expressing the amino acid sequence is disclosed.

## Description

The present invention relates to the use of peptide or protein, comprising an amino acid domain, chosen from the group, consisting of the following amino acid sequences: KRLFKKLKFSLRKY, KRLFKKLLFSLRKY, LLLFLLKKRKKRKY, and to peptides or proteins, comprising an amino acid domain, sharing at least 40% identity, or more with any of the above-mentioned amino acid sequences. Said sequences are listed from the amino terminal on the left to the carboxyl terminal to the right.

In WO99/37678, herein incorporated by reference, peptides of the above-mentioned type are described and identified as antimicrobial peptides which can be used against antimicrobial infections.

In WO00/01427, the use of the above-identified peptides in bone cement is disclosed.

The above-mentioned amino acid domains are derived from saliva of human origin and are used for administration into the human body. Because these peptides are of human origin, they have a low activity against human erythrocytes and have therefore a low toxicity in humans. The use in the human body is only recommended because of the above-mentioned low toxicity compared to antimicrobial peptides of non-human origin. However, the use of said human derived peptides for applications outside the human body is not recommended in the art, because of the relatively low activity of these peptides. Therefore, it is recommended in the art to use antimicrobial peptides of non-human origin for applications outside the human body, as the antimicrobial activity of non-human peptides has been believed to be substantially higher than that of human origin.

The present inventors suprisingly found that peptides or proteins, comprising an amino acid domain, chosen from the group, consisting of the following amino acid sequences: KRLFKKLKFSLRKY, KRLFKKLLFSLRKY, LLLFLLKKRKKRKY, can very advantageously be used as active ingredient in an antimicrobial preparation for surface treatment of articles to counteract microbial growth on the said surface.

The peptide or protein may also comprise an amino acid domain sharing at least 40% identity, more preferably at least 60%, more preferably at least 80% and most preferably at least 90% sequence identity with one of the above amino acid sequences, wherein sequence identity is defined as the percentage of identical matches between a stretch of 20 amino acids or less, preferably 16 amino acids or less, most preferably 14 amino acids of a peptide or protein, and one of the above-mentioned amino acid sequences.

The antimicrobial preparation according to the invention can be in liquid form which can e.g. easily be sprayed on the surface to be treated. It has been shown that growth of bacteria, fungi and yeasts can effectively be counteracted by treatment of the surface with the antimicrobial preparation according to the invention.

Although the antimicrobial preparation may comprise as active ingredient a protein comprising one or more of the above-mentioned amino acid domains, e.g. in the form of a fusion protein, produced by genetic engineering techniques, it is preferred that the active ingredient is present in the form of a peptide of less than 100 amino acids, preferably less than 70 amino acids, more preferably less than 50 amino acids, and most preferably has between 14 and 25 amino acids. These small peptides have been shown to be very active antimicrobial agents.

In another advantageous embodiment the peptide or protein is applied to the surface of food, in particularly fresh food, more in particular fresh meat of fish. It has been shown that bacterial spoilage can effectively be counteracted by e.g. spraying a liquid preparation comprising one or more of the above-identified peptides or proteins onto the surface of said food products.

In a preferred embodiment, the peptides or proteins are used for application onto the surface of medical devices, such as surgery instruments, contactlenses etc. For this, the above identified peptides or proteins can be incorporated in a desinfection spray or in contactlens liquids. In other preferred embodiments, the peptide or protein as above defined can be used as additive for food and for products, including animal food and animal food products, hygienic health care products, such a contactlens solutions, lotions, soap, etc., in desinfectants and cleaning detergents. In e.g. animal food and animal food products, the peptide or protein can be incorporated in solid or liquid form.

It is also advantageous to use the present peptides or proteins as active antimicrobial ingredient in a biocide. A biocide of this type can be designed such that it is completely biological, i.e. without any toxic synthetic chemicals, which biocide will be easily biodegradable.

The invention further relates to an antimicrobial preparation for surface treatment of articles or as active antimicrobial additive in food, hygienic health care products, desinfectants, cleaning detergents, comprising a peptide or protein, comprising an amino acid domain, sharing at least 40% identity, preferably at least 60%, more preferably at least 80% and most preferably at least 90% sequence identity with an amino acid sequence, chosen from the group, consisting of:

KRLFKKLKFSLRKY,

KRLFKKLLFSLRKY,

LLLFLLKKRKKRKY.

Preferably the antimicrobial preparation comprises a peptide or protein comprising an amino acid domain that is identical to one of the above listed amino acid sequences.

The preparation, especially for surface treatment of articles, is preferably in liquid form and comprises 1µM-1µM of the peptide or protein in a suitable buffer. A suitable buffer can be any buffer, wherein the peptide or proteins are in a "natural" active form. Examples of such buffers are phosphate buffered saline (PBS) and TE-buffer. The solution can also comprise salts, like NaCl, KCl and the like.

In a further attractive embodiment, the invention relates to a method for avoiding microbial spoilage of human or animal food, in particular fresh meat or fish, comprising the step of applying onto the surface of the food the antimicrobial preparation according to the invention. Preferably, the preparation is applied onto the food surface in such an amount, that the food product comprises 2,5-25 mg antimicrobial peptide or protein from the preparation per kg of the food product. When the peptide or protein is incorporated in food, such as animal food, said food also comprises preferably 2,5-25 mg of the peptide/protein per kg of the food.

Another very attractive embodiment of the present invention relates to a transgenic plant, comprising cells, which cells comprise a DNA sequence, expressable in said cells, encoding a peptide or protein, comprising an amino acid domain, chosen from the group, consisting of the following amino acid sequences:

KRLFKKLKFSLRKY,

KRLFKKLLFSLRKY,

LLLFLLKKRKKRKY.

"Expressable" means here that the DNA sequence (the "transgenic sequence") is present in the plant cells, such that an amino acid sequence encoded by the said DNA sequence is produced by the said plant cells. Genetic elements, necessary for the proper expression of the DNA sequence and peptide production must be available for the said plant cells, and are thereto either present on or encoded by the plant cell genome or the transgenic sequence.

According to the invention, said peptide or protein may also comprise an amino acid domain sharing at least 40% identity, preferably at least 60%, more preferably at least 80% and most preferably at least 90% sequence identity with one of the above amino acid sequences, although a domain, identical to one of these sequences is preferred. As the peptide or proteins will have antimicrobial activity because of the presence of the above identified amino acid domain, the transgenic plants may express a DNA sequence, leading to the production and presence of the active antibiotic peptides or proteins in the transgenic plant cells. The presence of said peptides or proteins confers microbial resistance to the plants.

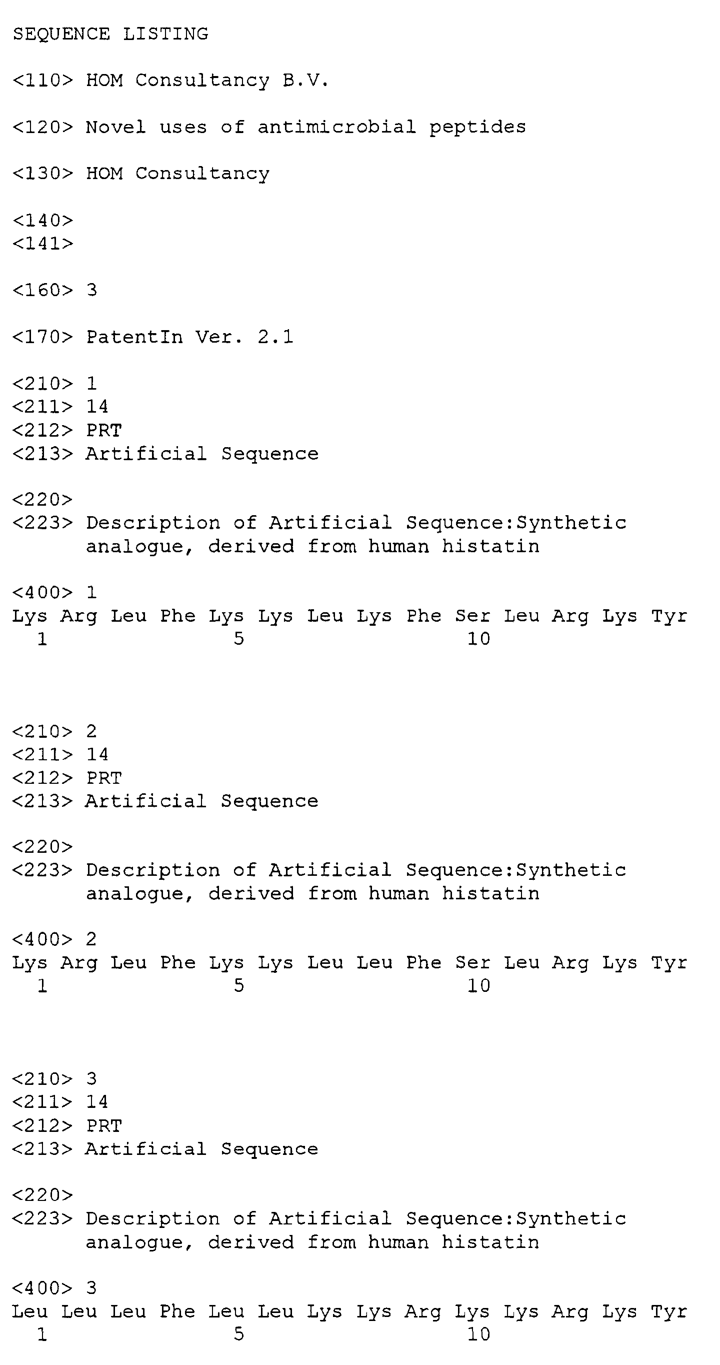

## Claims

1. Use of a peptide or protein, comprising an amino acid domain, chosen from the group, consisting of the following amino acid sequences: KRLFKKLKFSLRKY, KRLFKKLLFSLRKY, LLLFLLKKRKKRKY, as active antimicrobial ingredient in an antimicrobial preparation for surface treatment of articles to counteract microbial growth on the said surface.

2. Use of a peptide or protein, comprising an amino acid domain, sharing at least 40%, preferably at least 60%, more preferably at least 80% and most preferably at least 90% sequence identity with an amino acid sequence, chosen from the group, consisting of the following amino acid sequences: KRLFKKLKFSLRKY, KRLFKKLLFSLRKY, LLLFLLKKRKKRKY, as active antimicrobial ingredient in an antimicrobial preparation for surface treatment of articles to counteract microbial growth on the said surface.

3. Use according to claim 1 or 2, wherein the peptide has a length of less than 100 amino acids, preferably less than 70 amino acids, more preferably less than 50 amino acids, most preferably 14-25 amino acids or less.

4. Use according to any of the preceding claims for application onto the surface of medical devices.

5. Use according to any of the preceding claims for application onto the surface of food, in particular fresh food, more in particular fresh meat or fish.

6. Use of a peptide or protein as defined in any of the preceding claims as antimicrobial additive for food and food products, including animal food and animal food products.

7. Use of a peptide or protein as defined in any of the preceding claims as active antimicrobial additive in hygienic health care products, desinfectants, cleaning detergents.

8. Use of a peptide or protein as defined in any of the preceding claims as active antimicrobial additive in a biocide.

9. Antimicrobial preparation for surface treatment of articles or as active antimicrobial additive in food, in hygienic health care products, desinfectants, cleaning detergents or as biocide, comprising a peptide or protein, comprising an amino acid domain, sharing at least 40% identity, preferably at least 60%, more preferably at least 80% and most preferably at least 90% sequence identity with an amino acid sequence, chosen from the group, consisting of the following amino acid sequences:

KRLFKKLKFSLRKY,

KRLFKKLLFSLRKY,

LLLFLLKKRKKRKY.

10. Antimicrobial preparation according to claim 9, the peptide or protein comprising an amino acid domain, chosen from the group, consisting of the following amino acid sequences:

KRLFKKLKFSLRKY,

KRLFKKLLFSLRKY,

LLLFLLKKRKKRKY.

11. Preparation according to claim 9 or 10, wherein the peptide has a length of less than 100 amino acids, preferably less than 70 amino acids, more preferably less than 50 amino acids, most preferably between 14 and 25 amino acids.

12. Preparation according to any of claims 9-11, comprising 1µM-1mM of the peptide or protein in a suitable buffer.

13. Method for avoiding microbial spoilage of human or animal food, in particular fresh meat or fish, comprising the step of applying onto the surface of the food the antimicrobial preparation according to any of the claims 9-12.

14. Food product, comprising 2,5-25 mg antimicrobial peptide or protein from the preparation per kg of the food product.

15. Transgenic plant, comprising cells that comprise a DNA sequence encoding a peptide or protein, expressible in the said plants, the said peptide or protein comprising an amino acid domain, chosen from the group, consisting of the following amino acid sequences:

KRLFKKLKFSLRKY,

KRLFKKLLFSLRKY,

LLLFLLKKRKKRKY.

16. Transgenic plant, comprising cells that comprise a DNA sequence encoding a peptide or protein, expressible in the said plants, the said peptide or protein comprising an amino acid domain, sharing at least 40%, preferably at least 60%, more preferably at least 80% and most preferably at least 90% sequence identity with an amino acid sequence, chosen from the group, consisting of the following amino acid sequences:
